# EUROPÄISCHE PATENTSCHRIFT

(11) **EP 2 146 949 B1**
(45) Veröffentlichungstag und Bekanntmachung des Hinweises auf die Patenterteilung: **01.03.2017**
(21) Anmeldenummer: 08736633.2
(22) Anmeldetag: 30.04.2008
(51) Int. Cl.: C07C 59/125, C07C 211/63, C07C 51/367, C07C 51/41, B01F 17/44

(54) **IONISCHE FLÜSSIGKEITEN MIT POLYETHERCARBOXYLATEN ALS ANIONEN, DEREN HERSTELLUNG UND VERWENDUNG**
IONIC LIQUIDS COMPRISING POLYETHERCARBOXYLATES AS ANIONS, PRODUCTION AND USE THEREOF
LIQUIDES IONIQUES AVEC POLYETHERCARBOXYLATES COMME ANIONS, LEUR FABRICATION ET LEUR UTILISATION

(30) Priorität: 05.05.2007 DE 102007021197; 19.12.2007 EP 07123577
(43) Veröffentlichungstag der Anmeldung: 27.01.2010
(73) Patentinhaber: BASF SE, 67056 Ludwigshafen am Rhein (DE)
(72) Erfinder: KUNZ, Werner, 93342 Saal (DE); THOMAIER, Stefan, 93059 Regensburg (DE); MAURER, Eva, 94469 Deggendorf (DE); ZECH, Oliver, 93047 Regensburg (DE); KELLERMEIER, Matthias, 93336 Altmannstein (DE); KLEIN, Regina, 93051 Regensburg (DE)
(74) Vertreter: BASF IP Association
(86) Internationale Anmeldenummer: PCT/EP2008/055314
(87) Internationale Veröffentlichungsnummer: WO 2008/135482

(56) Entgegenhaltungen:
- EP-A- 0 716 072
- WO-A-02/24623
- WO-A-97/28238
- WO-A-2005/070896
- DE-A1- 4 225 626
- DE-C1- 19 928 128
- US-A- 5 233 087
- R. D. ROGER, K. R. SEDDON: "Ionic Liquids - Solvents of the Future?" SCIENCE, Bd. 302, 31. Oktober 2003 (2003-10-31), Seiten 792-793, XP002494444

## Beschreibung

Die vorliegende Erfindung betrifft neuartige ionische Flüssigkeiten, welche als Anionen Polyethercarboxylate enthalten, ein Verfahren zu deren Herstellung sowie deren Verwendung.

Unter ionischen Flüssigkeiten versteht der Fachmann Flüssigkeiten, welche ausschließlich aus Ionen bestehen und einen Schmelzpunkt von weniger als 100°C aufweisen. Beispiele typischer Kationen in ionischen Flüssigkeiten umfassen Tetraalkylammonium-, Tetraalkylphosphonium-, Dialkylimidazolium- oder Alkylpyridiniumkationen und Beispiele typischer Anionen umfassen Trifluormethansulfonat, Tetrafluoroborat, Alkylsulfonate oder Bis(trifluormethylsulfonyl)imid). Ionische Flüssigkeiten haben in den letzten Jahren zunehmend an Bedeutung gewonnen. Sie sind beispielsweise zur Verwendung als Lösungsmittel, als Wärmeträger, als Elektrolyt in Batterien, in Doppelschicht-Kondensatoren, in Solarzellen oder zur Verwendung in Extraktions- oder Trennprozessen vorgeschlagen worden. Ein Überblick über ionische Flüssigkeiten findet sich beispielsweise in P. Wasserscheid, W. Keim, Angew. Chem. 2000, 112, 3926 bis 3945 oder in *"*Ionic Liquids", Ullmann's Encyclopedia of Industrial Chemistry, Electronic Release, 7th Edition, Wiley-VCH, Weinheim, New York 2007.

WO 2005/070896 und WO 2007/057403 offenbaren die Verwendung von Anionen R^{a}-S⁻ als Anionen in ionischen Flüssigkeiten. Hierbei kann es sich bei S⁻ um verschiedene Säuregruppen wie beispielsweise Sulfonate, Sulfite, Phosphonate, Phosphite oder Carboxylgruppen handeln. Bei dem Rest R^{a} kann es sich um eine Vielzahl verschiedenster, ggf. substituierter Kohlenwasserstoffreste handeln, darunter auch um Alkylether- oder Alkylpolyethergruppen. Als Reste R werden konkret 5-Methoxy-3-oxapentyl, 8-Methoxy-3,6-dioxa-octyl, 11-Methoxy-3,6,9-trioxaundecyl, 7-Methoxy-4-oxaheptyl, 11-Methoxy-4,8-dioxa-undecyl, 15-Methoxy-4,8,12-trioxapentadecyl, 9-Methoxy-5-oxanonyl, 14-Methoxy-5,10-oxatetradecyl sowie die entsprechenden Ethoxy- und Hydroxyderivate offenbart. Spezielle Kombinationen dieser Reste mit bestimmten Säuregruppen und bestimmten Kationen werden jedoch nicht offenbart.

US 5,233,087 offenbart Alkylpolyethoxycarboxylate der allgemeinen Formel RO(CH₂CH₂O)ₓCH₂COO⁻ M⁺, wobei es sich bei R um einen C₈- bis C₁₈-Alkylrest, x eine Zahl von 1 bis 15 und bei M ein Alkalimetall- oder Erdalkalimetallion handelt. Die Alkylethoxycarboxalyte werden durch Umsetzen des entsprechenden Alkohols RO(CH₂CH₂O)ₓH mit Chloressigsäure erhalten. Sie werden als Tenside in wässriger Lösung verwendet.

EP 716 072 A1 offenbart konzentrierte, fließfähige Polyethercarboxylate der allgemeinen Formel RO(AO)ₙCH₂COO⁻ M⁺ wobei es sich bei R um einen C₈- bis C₂₄-Alkylrest oder einen alkylsubstituierten Arylrest mit 9 bis 24 C-Atomen, bei n eine Zahl von 1 bis 15, bei A um C₂- bis C₅-Alkylen und M um ein Alkalimetall-, ½ Erdalkali-, Ammonium - oder ein substituiertes Ammoniumion handelt. Sie können als Dispergier- und Emulgiermittel im Wasch- und Reinigungsmittelbereich eingesetzt werden.

Weitere Beispiele für Tenside auf Basis von Alkylpolyethercarboxylaten offenbaren WO 97/28238 oder DE 10 2004 027 329 A1. Tenside auf Basis von Alkylpolyethercarboxylaten sind auch kommerziell erhältlich, beispielsweise unter der Marke Akypo^{®}. Keine der genannten Schriften offenbart aber die Verwendung derartiger Alkylpolyethercarboxylate in ionischen Flüssigkeiten.

WO 2002/24623 beschreibt Polyethercarbonsäuren sowie deren Salze und Solvate. Geeignete Salze sind Alkalimetall Salze wie Natrium und Kalium und teritäre Alkylammoniumsalze wie *tert*-Butyl Ammonium. In den Beispielen werden 2,5,8,11-Tetraoxatridecan-13-oic acid sowie {2-[2-(2,2,3,3,3-Pentafluoropropoxy)ethoxy]-ethoxy}acetic acid beschrieben. Bei der Herstellung dieser Polyethercarbonsäuren werden wässrige Lösungen der entsprechende Natriumsalze als Zwischenprodukte gebildet. Die Polyethercarbonsäuren werden als Stabilisatoren in pharmazeutischen Aerosol Formulierungen eingesetzt.

DE19928128 beschreibt Ethercarbonsäuren, welche als Detergenzien bekannt sind. Sie finden in Wasch- und Kosmetikformulierungen, aber auch in technischen Anwendungen Verwendung. In den Beispielen werden Isononylalkohol und Oleylalkohol als Alkohole bei der Herstellung von Ethercarbonsäuren eingesetzt.

DE 4225626 beschreibt die Verwendung von Ethercarbonsäuren der allgemeinen Formel R-(OC₂H₄)ₙ-OCH₂COOH mit R etwa C₄-C₁₀-alkyl und n etwa 2-8 als keimtötenden Substanzen in wässrigen sauren Medien aus anorganischen oder organischen Säuren oder deren Mischungen.

Roger, R.D.; Seddon, K.R. Science, 302, 2003, 792-793 beschreibt die Verwendung von ionischen Flüssigkeiten allgemein als Lösungsmittel.

Die Tatsache, dass ionische Flüssigkeiten nur aus Kationen und Anionen bestehen, erklärt ihre einzigartigen Eigenschaften. Sie besitzen u.a. einen sehr niedrigen Dampfdruck, können eine Vielzahl organischer und anorganischer Materialien lösen und zeichnen sich durch eine hohe Temperaturstabilität aus. Die physikalischen und chemischen Eigenschaften ionischer Flüssigkeiten, wie beispielsweise Schmelzpunkt, thermische Stabilität, Viskosität, Leitfähigkeit, Solvatationsstärke, Löslichkeitseigenschaften, Acidität und Koordinationsfähigkeit werden durch die verwendeten Ionen bestimmt, d.h. sie lassen sich durch die Auswahl von Kation und Anion in einem weiten Bereich gezielt variieren und für die jeweilige Verwendung anpassen.

Ionische Flüssigkeiten sind mittlerweile auch kommerziell erhältlich. Um für jeden Prozess die passende ionische Flüssigkeit zur Verfügung zu haben, ist es von großem Interesse, neue ionische Flüssigkeiten zu synthetisieren. Aufgabe der Erfindung war es daher, neuartige ionische Flüssigkeiten zur Verfügung zu stellen.

Dementsprechend wurden ionische Flüssigkeit der allgemeinen Formel 1/n[Xn⁺] [Y⁻] mit einem Schmelzpunkt von weniger als 100°C gefunden, wobei n für 1,2 oder 3 steht, und wobei
- es sich bei dem Kation Xⁿ⁺ um ein Metallion,
- das Anion Y⁻ die allgemeine Formel (II) aufweist

   R⁶O-(R⁷-O-)ₓ-R⁸-COO⁻ (II),

   wobei
   - x für eine Zahl von 2 bis 8 steht,
   - R⁶ für eine verzweigte oder unverzweigte Kohlenwasserstoffgruppe mit 1 bis 3 Kohlenstoffatomen steht,
   - die Reste R⁷ jeweils unabhängig voneinander für eine verzweigte oder unverzweigte Alkylengruppe mit 2 bis 4 Kohlenstoffatomen stehen, und
   - R⁸ für eine verzweigte oder unverzweigte Alkylengruppe mit 1 bis 4 Kohlenstoffatomen steht,
und wobei in den Resten R⁶, R⁷ und R⁸ jeweils die Wasserstoffatome auch ganz oder teilweise durch Fluor substituiert sein können.

Zu der Erfindung ist im Einzelnen das Folgende auszuführen:
Unter "Ionischen Flüssigkeiten" sollen nachfolgend im üblichen Sinne lösemittelfreie, insbesondere wasserfreie Zusammensetzungen mit einem Schmelzpunkt von weniger als 100°C verstanden werden, welche ausschließlich aus Kationen und Anionen zusammengesetzt sind.

Die erfindungsgemäßen ionischen Flüssigkeiten weisen die allgemeine Formel 1/n[Xⁿ⁺] [Y⁻] auf.

Bei den Kationen Xⁿ⁺ steht die Zahl n für 1, 2 oder 3, bevorzugt 1 oder 2 und besonders bevorzugt für 1.

Bei den Kationen handelt es sich um Metallionen, insbesondere um Alkalimetall- oder Erdalkalimetallionen. Beispiele bevorzugter Metallionen umfassen Li⁺, Na⁺, K⁺, Be²⁺, Mg²⁺ und Ca²⁺. Besonders bevorzugt sind Na⁺ und K⁺ und ganz besonders bevorzugt ist Na⁺.

Das Anion Y^{m-} weist erfindungsgemäß die allgemeine Formel (II)

R⁶O-(R⁷-O-)ₓ-R⁸-COO⁻ (II)

auf. Bei dem Rest R⁶ handelt es sich um eine geradkettige oder verzweigte Kohlenwasserstoffgruppe mit 1 bis 3 C-Atomen, wobei die Wasserstoffatome auch ganz oder teilweise durch Fluor substituiert sein können. Bevorzugt handelt es sich um einen Alkylrest ausgewählt aus der Gruppe von Methyl, Ethyl, n-Propyl oder i-Propyl. Bevorzugt handelt es sich um eine Methyl- oder eine Ethylgruppe und ganz besonders bevorzugt um eine Methylgruppe.

Der Rest R⁶ ist über x Alkoxygruppen der Formel -R⁷-O- mit der Gruppe -R⁸-COO⁻verbunden, wobei die Alkoxygruppen in einem Anion gleich oder verschieden sein können.

Bei den Resten R⁷ handelt es sich um Alkylengruppen mit 2 bis 4 Kohlenstoffatomen, wobei die Wasserstoffatome auch ganz oder teilweise durch Fluor substituiert sein können. Beispiele für R⁷ umfassen 1,2-Ethylengruppen -CH₂-CH₂-, 1,2-Propylengruppen -CH₂-CH(CH₃)-, 1,3-Propylengruppen -CH₂-CH₂-CH₂-, 1,4-Butylengruppen -CH₂-CH₂-CH₂-CH₂- oder 1,2-Butylengruppen -CH₂-CH(C₂H₅)-. Bevorzugt handelt es sich um 1,2-Ethylengruppen -CH₂-CH₂- und/oder 1,2-Propylengruppen -CH₂-CH(CH₃)- und besonders bevorzugt um 1,2-Ethylengruppen -CH₂-CH₂-. Bevorzugt handelt es sich bei mindestens 50 % der Alkylengruppen um 1,2-Ethylengruppen. Sofern es sich um verzweigte Alkylengruppen handelt, können diese in der dargestellten Orientierung oder auch in umgekehrter Orientierung in das Anion eingebaut sein.

Bei dem Rest R⁸ handelt es sich um eine verzweigte oder unverzweigte Alkylengruppe mit 1 bis 4 Kohlenstoffatomen, wobei die Wasserstoffatome auch ganz oder teilweise durch Fluor substituiert sein können. Beispiele umfassen eine Methylengruppe -CH₂-, eine 1,2-Ethylengruppe -CH₂-CH₂-, eine 1,2-Propylengruppe -CH₂-CH(CH₃)-, eine 1,3-Propylen-gruppe -CH₂-CH₂-CH₂-, eine 1,4-Butylengruppe -CH₂-CH₂-CH₂-CH₂- oder eine t-Butylengruppe -CH₂-C(CH₃)₂-. Bevorzugt handelt es sich um eine Methylengruppe -CH₂-.

Die Zahl x weist einen Wert von 2 bis 8, bevorzugt 2 bis 6, besonders bevorzugt 3 bis 5 und ganz besonders 3 auf, wobei x hierbei in bekannter Art und Weise für den Mittelwert der vorhandenen Alkoxygruppen steht, welcher selbstverständlich nicht eine natürliche Zahl sein muss, sondern auch eine beliebige rationale Zahl sein kann.

Die Eigenschaften der erfindungsgemäßen ionischen Flüssigkeiten können vom Fachmann durch die Auswahl von Anionen und Kationen beeinflusst werden. Selbstverständlich kann eine ionische Flüssigkeit auch mehrere verschiedene Anionen Y⁻und/oder mehrere verschiedene Kationen Xⁿ⁺ enthalten.

Ohne dass wir hierbei an eine bestimmte Theorie gebunden sein möchten, scheinen die besonderen Eigenschaften der erfindungsgemäßen ionischen Flüssigkeiten durch Chelatbildung zumindest beeinflusst zu sein, wie nachfolgend exemplarisch für den Fall einer Carboxylgruppe und eines Natriumions gezeigt ist.

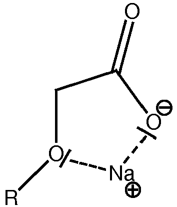

Durch diese interne Chelatbildung nimmt der salzartige Charakter der ionischen Flüssigkeiten ab und die ionische Flüssigkeit verhält sich unpolarer. Beispielsweise ist der Schmelzpunkt der erfindungsgemäßen ionischen Flüssigkeiten im Vergleich zu anderen Salzen mit ähnlichen Kationen relativ niedrig und das Lösevermögen für unpolare Substanzen ist gut.

Besonders bevorzugt sind ionische Flüssigkeiten aus dem Anion H₃CO-(CH₂-CH₂-O)₃-CH₂-COO⁻ (III) und Kationen ausgewählt aus der Gruppe von Na⁺ und K⁺.

Weiterhin besonders bevorzugte Kationen für das Anion (III) sind Ca²⁺ und Mg²⁺.

Die Herstellung der erfindungsgemäßen ionischen Flüssigkeiten kann beispielsweise erfolgen, indem man zunächst einmal die dem Anion R⁶O-(R⁷-O-)ₓ-R⁸-COO⁻ (II) entsprechenden Säuren R⁶O-(R⁷-O-)ₓ-R⁸-COOH (V) synthetisiert und in einem zweiten Schritt die Säure mit einer Base umsetzt, welche das Kation oder eine Vorstufe davon enthält. Eventuell im Zuge der Synthese verwendete Lösemittel werden aus dem Reaktionssystem entfernt.

Zur Synthese der Säuren R⁶O-(R⁷-O-)ₓ-R⁸-COOH (V) kann beispielsweise man von den entsprechenden Polyoxyalkylenmonoalkylethern R⁶O-(R⁷-O-)ₓ-H (IV) ausgehen. Diese werden mit einer Halogencarbonsäure der allgemeinen Formel Hal-R⁸-COOH zur Carbonsäure R⁶O-(R⁷-O-)ₓ-R⁸-COOH (V) umgesetzt, wobei Hal für F, Cl, Br und I, bevorzugt Cl, Br und I steht. Bevorzugt kann eine ω-Chlorcarbonsäure der allgemeinen Formel Cl-R⁸-COOH eingesetzt werden, und in einer besonders bevorzugten Synthesevariante setzt man Chloressigsäure ein. Die Umsetzung kann vorgenommen werden, indem man den wasserfreien Etheralkohol (IV) mit elementarem Natrium zum reaktiveren Alkoholat umsetzt. Die Carbonsäure (V) kann mittels dem Fachmann bekannter Technologien isoliert werden und beispielsweise durch Vakuumdestillation gereinigt werden.

Die Carbonsäuren (V) können in einem zweiten Schritt mit entsprechenden Basen neutralisiert werden. Lösemittel, in denen die Base eventuell gelöst war, werden anschließend destillativ abgetrennt. Die Herstellung des Natriumsalzes kann beispielsweise durch Neutralisation mit der entsprechenden Menge wässriger NaOH, gefolgt vom Entfernen des Wassers im Vakuum erfolgen. Sie kann auch wasserfrei durch Umsetzen der Carbonsäure (V) mit Natriumhydrogencarbonat in einem Alkohol gefolgt vom destillativen Abtrennen des Alkohols erfolgen.

Die erfindungsgemäßen ionischen Flüssigkeiten weisen einen Schmelzpunkt von weniger als 100°C, bevorzugt weniger als 75°C und besonders bevorzugt weniger als 60°C auf. Der Schmelzpunkt kann vom Fachmann durch die Wahl entsprechender Anionen und Kationen je nach dem gewünschten Schmelzpunkt festgelegt werden. Es lassen sich auch leicht bei Raumtemperatur flüssige ionische Flüssigkeiten bereitstellen.

Die erfindungsgemäßen ionischen Flüssigkeiten können insbesondere als Löse- oder Extraktionsmittel verwendet werden.

Sie unterscheiden sich von anderen ionischen Flüssigkeiten durch eine stärkere Fähigkeit, Kationen zu komplexieren. Sie enthalten weiterhin keine toxischen Komponenten und weisen eine sehr hohe elektrochemische Stabilität auf. Die erfindungsgemäßen ionischen Flüssigkeiten eignen sich besonders als Lösemittel zur Durchführung chemischer Reaktionen, insbesondere zur Durchführung von Metathesereaktionen oder als Lösemittel für natürliche und synthetische Polymere. Dabei zeigen sie je nach Zusammensetzung die Eigenschaften von klassischen Lösungsmitteln, von Hydrotropen oder von Tensiden.

Sie können weiterhin als Extraktionsmittel für die flüssig-flüssig-Extraktion verwendet werden, beispielsweise zur Extraktion von Schwermetallen, pharmakologisch aktiven Substanzen, Naturstoffen, Nahrungsstoffen bzw. Nahrungszusatzstoffen.

Sie können weiterhin als nichtwässrige Elektrolyte oder Komponenten nichtwässriger Elektrolyte in Energiespeichersystemen, beispielsweise Batterien, oder Energieumwandlungssystemen, wie beispielsweise Solarzellen eingesetzt werden. Außerdem eignen sie sich als Lösungsmittel für elektrochemische Prozesse, wie beispielsweise elektrochemische Metallabscheidung.

Weitere Verwendungen umfassen die Verwendung als Lösemittel und/oder strukturierende Matrix in der Nanopartikelsynthese, die Verwendung zum Stabilisieren von Nanopartikeln oder als Lösemittel für natürliche und synthetische Polymere.

Selbstverständlich können die erfindungsgemäßen ionischen Flüssigkeiten zum Einsatz auch mit anderen ionischen Flüssigkeiten gemischt werden. Hierdurch lassen sich weitere ionische Flüssigkeiten mit neuartigem Eigenschaftsspektrum erhalten.

Die erfindungsgemäßen ionischen Flüssigkeiten haben bei der Verwendung als Lösungs- und Extraktionsmittel eine Reihe von Vorteilen:
Die ionische Flüssigkeit erlaubt es, Lösungen von Salzen in einem nicht-wässrigen, aprotischen Medium zur Verfügung zu stellen. Solche Lösungen lassen sich in elektrochemischen Verfahren, beispielsweise der elektrochemischen Metallabscheidung vorteilhaft nutzen, denn nicht jedes Metall lässt sich aus einem wässrigen Medium abscheiden. Das Cyclovoltagramm der erfindungsgemäßen ionischen Flüssigkeiten zeigt ein breites Spannungsfeld und somit eine hohe Redox-Stabilität. Sie sind daher für elektrochemische Anwendungen besonders gut geeignet.

Durch ihren aprotischen Charakter sind die ionischen Flüssigkeiten als Reaktionsmedium für chemische Reaktionen geeignet, in deren Verlauf salzartige oder polare Stoffe in Lösung gehalten werden müssen, die aber gleichzeitig durch das Vorhandensein von protischen Gruppen, wie beispielsweise OH-Gruppen oder Wasser gestört würden.

Ferner weisen ionischen Flüssigkeiten elektrische Leitfähigkeit auf, ohne Wasser zu enthalten und kommt dadurch für eine Vielzahl technischer Verfahren Frage, in denen wasserfreie Flüssigkeiten mit hoher Dielektrizitätskonstante eingesetzt werden müssen.

Die erfindungsgemäßen ionischen Flüssigkeiten sind schwer flüchtig. Schwer flüchtige Lösemittel sind in technische Anlagen interessant, in denen es auf die Erzielung niedriger Drucke ankommt (z. B. Pumpen). Weiterhin weisen insbesondere die Na⁺ und K⁺-Salze sehr hohe Zersetzungspunkte und eine geringe Korrosivität auf. Sie eignen sich daher besonders für Hochtemperaturanwendungen, z.B. zur Hochtemperaturschmierung oder bei der Leiterplattenherstellung.

Die folgenden Beispiele sollen die Erfindung näher illustrieren:

### Beispiel 1 - Synthese von Natrium-2,5,8,11-tetraoxatridecan-13-oat (TEGMECH2COO⁻Na⁺)

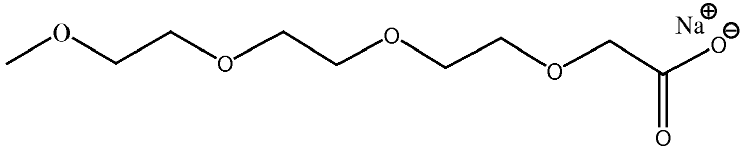

### 1. Stufe Herstellung der Polyethercarbonsäure:

15.3 g Na werden in 260 mL Triethylenglykolmonomethylether in Schutzgasatmosphäre unter starkem Rühren gelöst. Zunächst wird dabei bei Raumtemperatur gearbeitet und später sukzessive ca. 120°C erhitzt. Nach vollständigem Auflösen des Na wird auf 100°C abgekühlt, und es werden 30.9 g Chloressigsäure gelöst in 60 mL Triethylenglykol-monomethylether zugetropft. Die Reaktionsmischung wird daraufhin für etwa 12 h bei 100°C gerührt.

Überschüssiger Triethylenglykolmonomethylether wird nun im Ölpumpenvakuum (etwa 10⁻³ mbar) abdestilliert (Siedepunkt: ca. 88-90°C). Der erkaltete Rückstand wird anschließend mit ca. 150 mL Wasser und nach kurzem Rühren mit 23 mL 85%iger Phosphorsäure versetzt. Die nun klare braune Lösung wird insgesamt 3 x mit 200 mL Dichlormethan extrahiert. Die vereinigten organischen Phasen werden mit Magnesiumsulfat getrocknet. Filtration und Abziehen des Lösungsmittels liefern schließlich eine dunkelbraune ölige Flüssigkeit.

Die rohe 2,5,8,11-Tetraoxatridecan-13-säure wird durch zweifache Destillation im Öldiffusionspumpenvakuum (ca. 10⁻⁷ mbar; Siedepunkt 135-145°C) aufgereinigt. Letztlich erhält man eine farblose viskose Flüssigkeit.

### 2. Stufe: Umsetzung zum Na-Salz

Das Natriumsalz der 2,5,8,11-Tetraoxatridecan-13-säure wird durch Umsetzung der Säure mit äquimolaren Mengen an wässriger 0,1 m Natriumhydroxidlösung erhalten. Nach der Neutralisation wird das Wasser abrotiert. Trocknung des Produkts erfolgt sukzessive im Ölpumpen-, Öldiffusionspumpen- und Turbomolekularpumpenvakuum (jeweils mehrere Tage).

Alternativ kann die Verbindung Natrium-2,5,8,11-tetraoxatridecan-13-oat auch nichtwässrig durch Umsetzung von äquimolaren Mengen an 2,5,8,11-Tetraoxatridecan-13-säure und Natriumhydrogencarbonat in Ethanol hergestellt werden. Die Trocknung erfolgt analog zur wässrigen Synthese.

In einer dritten Variante kann die Polyethercarbonsäure in wässriger Lösung mit NaOH-Maßlösung gerade bis zum Äquivalenzpunkt titriert (über pH-Messung). Diese Methode vermeidet Ungenauigkeiten beim Einwiegen der beiden Komponenten, so dass Äquimolarität sicher gewährleistet ist. Die Aufarbeitung erfolgt wie oben beschrieben.

Natrium-2,5,8,11-tetraoxatridecan-13-oat ist mit Wasser, Dichlormethan, Pentanol und Aceton in jedem Verhältnis mischbar, mit Diethylether teilweise. Die Viskosität bei 25°C liegt bei niedriger Scherrate in der Größenordnung von einer Million Millipascalsekunden. Der Schmelzpunkt ist in Tabelle 1 aufgeführt.

### Beispiel 2: Synthese von Lithium-2,5,8,11-tetraoxatridecan-13-oat (TEGMECH2COO⁻Li⁺)

Es wird wie unter Beispiel 1 beschrieben vorgegangen, nur wurde zum Neutralisieren LiOH verwendet. Der Schmelzpunkt ist in Tabelle 1 aufgeführt.

### Beispiel 3: Synthese von Kalium-2,5,8,11-tetraoxatridecan-13-oat (TEGMECH2COO⁻K⁺)

Es wird wie unter Beispiel 2 beschrieben vorgegangen, nur wurde zum Neutralisieren KOH verwendet. Der Schmelzpunkt ist in Tabelle 1 aufgeführt.

### Beispiel 4: Synthese von Calcium-di(2,5,8,11-tetraoxatridecan-13-oat) (TEGMECH2COO⁻)₂ Ca²⁺)

Es wird wie unter Beispiel 2 beschrieben vorgegangen, nur wurde zum Neutralisieren CaCO₃ verwendet. Der Schmelzpunkt ist in Tabelle 1 aufgeführt.

### Beispiel 5 (Vergleichsbeispiel): Synthese von Hexylammonium-2,5,8,11-tetraoxatridecan-13-oat

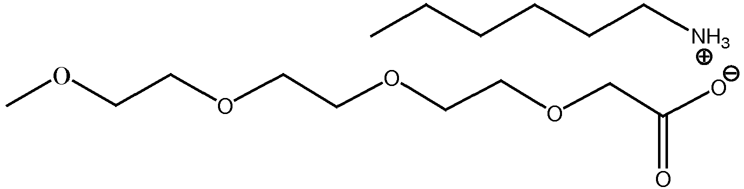

2,5,8,11-Tetraoxatridecan-13-säure wird wie in Beispiel 1 beschrieben hergestellt.

Die Alkylammoniumsalze sind generell durch direktes Umsetzen der Säure mit äquimolaren Mengen des betreffenden Amins zugänglich.

Zur Synthese von Hexylammonium-2,5,8,11-tetraoxatridecan-13-oat wird 2,5,8,11-Tetraoxatridecan-13-säure mit n-Hexylamin neutralisiert. Die Reaktion kann hierbei sowohl lösungsmittelfrei (z.B. Zutropfen von reinem Hexylamin zur Ethercarbonsäure, unter Kühlung) als auch in organischen Lösungsmitteln (typischerweise Ethanol) erfolgen. Um eine Rückreaktion und das Abdampfen des Amins während der Trocknung im Hochvakuum zu vermeiden, werden die Verbindungen lediglich im Ölpumpenvakuum etwa einen Tag lang getrocknet. Da die Synthesen wasserfrei erfolgen und da während der Neutralisation kein Wasser entsteht, reicht diese mildere Trocknung hier aus. Der Schmelzpunkt ist in Tabelle 1 aufgeführt.

Die Viskosität des Ammoniumsalzes ist deutlich niedriger als die des Natriumsalzes und liegt in der Größenordnung von 20.000 mPas. Sie ist ist damit vergleichbar mit der Viskosität von Honig oder Sirup. Aus elektrischen Leitfähigkeits- und Fluoreszenz-Messungen kann gefolgert werden, dass die ionische Flüssigkeit gemäß Beispiel 5 in wässriger Lösung hydrotropen Charakter aufweist.

### Beispiel 6 (Vergleichsbeispiel): Synthese von Dodecylammonium-2,5,8,11-tetraoxatridecan-13-oat

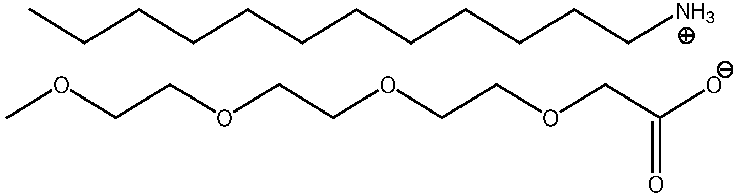

Es wird wie in Beispiel 4 vorgegangen, nur wird n-Dodecylamin zur Neutralisierung verwendet.

Aus Messungen der elektrischen Leitfähigkeit und der Oberflächenspannung im wässrigen Medium kann gefolgert werden, dass die ionische Flüssigkeit gemäß Beispiel 6 tensidischen Charakter aufweist. Aufgrund der amphiphilen und damit lösevermittelnden Eigenschaften weist die ionische Flüssigkeit gemäß Beispiel 6 ein breiteres Lösespektrum gegenüber herkömmlichen ionischen Flüssigkeiten auf. Weiterhin kann gefolgert werden, dass die ionische Flüssigkeit gemäß Beispiel 6 strukturierende Eigenschaften in Wasser, organischen Lösungsmitteln und insbesondere auch im Reinzustand besitzt. Dementsprechend kann sie beispielsweise als Strukturgeber (Templat) in Mineralisierungsprozessen dienen (z.B. für mesoporöse Silikate). Des Weiteren liegt die ionische Flüssigkeit aus Beispiel 6 bei Raumtemperatur flüssig vor (Schmelzpunkt siehe Tabelle 1). Aufgrund dieses sehr niedrigen Schmelzpunktes mischt sich die ionische Flüssigkeit mit Wasser in jedem Verhältnis zu einer niedrig viskosen Flüssigkeit im Gegensatz zu klassischen ionischen Tensiden, bei denen sich bei hohen Tensid-Konzentrationen in Wasser hochviskose flüssigkristalline Phasen ausbilden.

### Beispiele 7 bis 11 (Vergleichsbeispiele): Synthese von weiteren Alkylammonium-2,5,8,11-tetraoxatridecan-13-oaten

Es wurde wie in Beispiel 5 vorgegangen, nur wurden die folgenden Amine eingesetzt:
Beispiel 7 (Vergleichsbeispiel): n-Octylamin
Beispiel 8 (Vergleichsbeispiel): n-Decylamin
Beispiel 9 (Vergleichsbeispiel): n-Tetradecylamin
Beispiel 10 (Vergleichsbeispiel): n-Hexadecylamin
Beispiel 11 (Vergleichsbeispiel): n-Octadecylamin

Die Schmelzpunkte, Zersetzungspunkte und Viskositäten sind jeweils in Tabelle 1 aufgeführt.

### Zusammenstellung der Eigenschaften

**Tabelle 1: Schmelz- und Zersetzungspunkte (Einsetzen der Zersetzung) sowie Viskositäten verschiedener 2,5,8,11-tetraoxatridecan-13-oate.**

| | Kation | Schmelzpunkt [°C] | Zersetzungspunkt [°C] | Viskosität¹ [mPa · s] bei 25 °C |
|---|---|---|---|---|
| Beispiel 2 | Li⁺ | Glaspunkt < - 20 | n.b. | n.b. |
| Beispiel 1 | Na⁺ | Glaspunkt - 51.6 | 384 | 1.64 · 10⁵ |
| Beispiel 3 | K⁺ | 59.1 | 369 | n.b. |
| Beispiel 4 | Ca²⁺ | Glaspunkt - 80 | 320 | n.b. |
| Beispiel 5 (Vergleich) | n-Hexylammonium | Erstarrung bei -20 bis +16 | n.b. | 2.40 · 10² |
| Beispiel 7 (Vergleich) | n-Octylammonium | Erstarrung bei -20 bis +16 | 145 | 3.75 · 10² |
| Beispiel 8 (Vergleich) | n-Decylammonium | 14 | 156 | 4.00 · 10² |
| Beispiel 6 (Vergleich) | n-Dodecylammonium | 22 | 165 | 4.67 · 10² |
| Beispiel 9 (Vergleich) | n-Tetradecylammonium | 38 | 170 | n.b. |
| Beispiel 10 (Vergleich) | n-Hexadecylammonium | 47 | 175 | n.b. |
| Beispiel 11 (Vergleich) | n-Octadecylammonium | 55 | 182 | n.b. |

| | | | | |
|---|---|---|---|---|
| ¹Alle untersuchten Flüssigkeiten verhalten sich wie newtonsche Fluide n.b. nicht bestimmt | | | | |

Tabelle 1 zeigt, dass die Schmelzpunkte der ionischen Flüssigkeiten mit zunehmender Kettenlänge des Alkylrestes im Anion zunehmen. Durch Auswahl der Kettenlänge kann also der Schmelzpunkt gezielt eingestellt werden.

Ebenso lassen sich der Zersetzungspunkt und die Viskosität durch die Wahl des Kations beeinflussen.

### Verwendung der ionischen Flüssigkeiten

Mit dem gemäß Beispiel 1 synthetisierten Natrium-2,5,8,11-tetraoxatridecan-13-oat wurden Löseversuche durchgeführt.

Ammoniumnitrat ist in TEGMECH₂COONa auch bei Raumtemperatur gut löslich, während NaCl sich in geringen Konzentrationen bei Temperaturen über 100°C löst. Bei 120°C ist Magnesiumsulfat in TEGMECH₂COONa nennenswert löslich. Besonders erwähnenswert im Hinblick auf die Umweltverträglichkeit des Natriumsalzes ist die Löslichkeit von Olivenöl mit bis zu zehn Gewichtsprozent in TEGMECH2COONa.

## Patentansprüche

1. Ionische Flüssigkeit der allgemeinen Formel 1/n[Xⁿ⁺] [Y⁻] mit einem Schmelzpunkt von weniger als 100°C, wobei n für 1,2 oder 3 steht, **dadurch gekennzeichnet, dass**
• es sich bei dem Kation Xⁿ⁺ um ein Metallion handelt, und
• dass das Anion Y⁻ die allgemeine Formel (II) aufweist
R⁶O-(R⁷-O-)ₓ-R⁸-COO⁻ (II),
wobei
• x für eine Zahl von 2 bis 8 steht,
• R⁶ für eine verzweigte oder unverzweigte Kohlenwasserstoffgruppe mit 1 bis 3 Kohlenstoffatomen steht,
• die Reste R⁷ jeweils unabhängig voneinander für eine verzweigte oder unverzweigte Alkylengruppe mit 2 bis 4 Kohlenstoffatomen stehen, und
• R⁸ für eine verzweigte oder unverzweigte Alkylengruppe mit 1 bis 4 Kohlenstoffatomen steht,
wobei in den Resten R⁶, R⁷ und R⁸ jeweils die Wasserstoffatome auch ganz oder teilweise durch Fluor substituiert sein können.

2. Ionische Flüssigkeit gemäß Anspruch 1, **dadurch gekennzeichnet, dass** es sich bei dem Kation um mindestens eines ausgewählt aus der Gruppe von Alkalimetallionen und Erdalkalimetallionen handelt.

3. Ionische Flüssigkeit gemäß einem der Ansprüche 1 bis 2, **dadurch gekennzeichnet, dass** es sich bei R⁶ um eine Methylgruppe handelt.

4. Ionische Flüssigkeit gemäß einem der Ansprüche 1 bis 3, **dadurch gekennzeichnet, dass** es sich mindestens 50 % der Gruppen R⁷ um 1,2-Ethylengruppen handelt.

5. Ionische Flüssigkeit gemäß einem der Ansprüche 1 bis 4, **dadurch gekennzeichnet, dass** x für 3 bis 5 steht.

6. Ionische Flüssigkeit gemäß einem der Ansprüche 1 bis 5, **dadurch gekennzeichnet, dass** es sich bei R⁸ um eine Methylengruppe -CH₂- handelt.

7. Ionische Flüssigkeit gemäß Anspruch 1, **dadurch gekennzeichnet, dass** es sich bei dem Kation um Li⁺, Na⁺ oder K⁺, und es sich bei dem Anion um H₃CO-(-CH₂-CH₂-O-)₃-CH₂-COO⁻ handelt.

8. Verfahren zur Herstellung einer ionischen Flüssigkeit gemäß Anspruch 1,
**dadurch gekennzeichnet, dass** man
• in einer ersten Reaktionsstufe einen Polyoxyalkylenmonoalkylether R⁶O-(R⁷-O-)ₓ-H (IV) mit einer Halogencarbonsäure der allgemeinen Formel Hal-R⁸-COOH, wobei Hal für F, Cl, Br oder I steht, zur Carbonsäure R⁶O-(R⁷-O-)ₓ-R⁸-COOH (V) umsetzt, und
• in einer zweiten Reaktionsstufe die gebildete Carbonsäure R⁶O-(R⁷-O-)ₓ-R⁸-COOH (V) mit einer Base X(OH)ₙ oder [X - nH] unter Bildung der ionischen Flüssigkeit 1/n[Xⁿ⁺] [R⁶O-(R⁷-O-)ₓ-R⁸-COO⁻] neutralisiert,
mit der Maßgabe, dass verwendete Lösemittel und/oder während der Reaktion gebildete Lösemittel jeweils vollständig abgetrennt werden.

9. Verfahren gemäß Anspruch 8, **dadurch gekennzeichnet, dass** es sich bei der Halogencarbonsäure um Chloressigsäure handelt.

10. Verwendung von ionischen Flüssigkeiten gemäß einem der Ansprüche 1 bis 7 als Lösemittel.

11. Verwendung gemäß Anspruch 10, **dadurch gekennzeichnet, dass** man das Lösemittel zur Durchführung chemischer Reaktionen einsetzt.

12. Verwendung gemäß Anspruch 10, **dadurch gekennzeichnet, dass** man das Lösemittel zum Lösen natürlicher und synthetischer Polymere einsetzt.

13. Verwendung gemäß Anspruch 10, **dadurch gekennzeichnet, dass** man das Lösemittel für elektrochemische Prozesse einsetzt.

14. Verwendung gemäß Anspruch 10, **dadurch gekennzeichnet, dass** man das Lösemittel zum Herstellen und Stabilisieren von Nanopartikeln einsetzt.

15. Verwendung von ionischen Flüssigkeiten gemäß einem der Ansprüche 1 bis 7 als Extraktionsmittel für die flüssig-flüssig-Extraktion.

16. Verwendung von ionischen Flüssigkeiten gemäß einem der Ansprüche 1 bis 7 als Elektrolyte oder Komponenten nichtwässriger Elektrolyte in Energiespeichersystemen und Energieumwandlungssystemen,

17. Verwendung von ionischen Flüssigkeiten gemäß einem der Ansprüche 1 bis 7 als Hochtemperaturschmiermittel.

## Claims

1. An ionic liquid of the general formula 1/n[Xⁿ⁺] [Y⁻] having a melting point of less than 100°C, where n is 1, 2 or 3 and
• the cation Xⁿ⁺ is a metal ion, and
• the anion Y⁻ has the general formula (II)
R⁶O-(R⁷-O-)ₓ-R⁸-COO⁻ (II),
where
• x is from 2 to 8,
• R⁶ is a branched on unbranched hydrocarbon group having from 1 to 3 carbon atoms,
• the radicals R⁷ are each, independently of one another, a branched or unbranched alkylenic group having from 2 to 4 carbon atoms and
• R⁸ is a branched or unbranched alkylenic group having from 1 to 4 carbon atoms,
and the hydrogen atoms in the radicals R⁶, R⁷ and R⁸ can in each case be completely or partly replaced by fluorine.

2. The ionic liquid according to claim 1, wherein the cation is at least one cation selected from the group consisting of alkali metal ions and alkaline earth metal ions.

3. The ionic liquid according to any of claims 1 to 2, wherein R⁶ is a methyl group.

4. The ionic liquid according to any of claims 1 to 3, wherein at least 50% of the groups R⁷ are 1,2-ethylene groups.

5. The ionic liquid according to any of claims 1 to 4, wherein x is from 3 to 5.

6. The ionic liquid according to any of claims 1 to 5, wherein R⁸ is a methylene group -CH₂-.

7. The ionic liquid according to claim 1, wherein the cation is Li⁺, Na⁺ or K⁺, and the anion is H₃CO-(-CH₂-CH₂-O-)₃-CH₂-COO⁻.

8. A process for preparing an ionic liquid according to claim 1, wherein
• a polyoxyalkylene monoalkyl ether R⁶O-(R⁷-O-)ₓ-H (IV) is reacted with a halocarboxylic acid of the general formula Hal-R⁸-COOH, where Hal is F, Cl, Br or I, in a first reaction step to form the carboxylic acid R⁶O-(R⁷-O-)ₓ-R⁸-COOH (V) and
• the carboxylic acid R⁶O-(R⁷-O-)ₓ-R⁸-COOH (V) formed is neutralized with a base X(OH)ₙ or [X - nH] in a second reaction step to form the ionic liquid 1/n[Xⁿ⁺] [R⁶O-(R⁷-O-)ₓ-R⁸-COO⁻],
with the proviso that solvents used and/or solvents formed during the reaction are separated off completely.

9. The process according to claim 8, wherein the halocarboxylic acid is chloroacetic acid.

10. The use of ionic liquids according to any of claims 1 to 7 as solvents.

11. The use according to claim 10, wherein the solvent is used for carrying out chemical reactions.

12. The use according to claim 10, wherein the solvent is used for dissolving natural and synthetic polymers.

13. The use according to claim 10, wherein the solvent is used for electrochemical processes.

14. The use according to claim 10, wherein the solvent is used for producing and stabilizing nanoparticles.

15. The use of ionic liquids according to any of claims 1 to 7 as extractants for liquid-liquid extraction.

16. The use of ionic liquids according to any of claims 1 to 7 as electrolytes or components of nonaqueous electrolytes in energy storage systems and energy conversion systems.

17. The use of ionic liquids according to any of claims 1 to 7 as high-temperature lubricants.

## Revendications

1. Liquide ionique de formule générale 1/n[Xⁿ⁺][Y⁻] ayant un point de fusion inférieur à 100 °C, n représentant 1, 2 ou 3, **caractérisé en ce que**
- le cation Xⁿ⁺ est un ion métallique, et
- l'anion Y⁻ présente la formule générale (II)
R⁶O-(R⁷-O-)ₓ-R⁸-COO⁻ (II),
dans laquelle
- x représente un nombre de 2 à 8,
- R⁶ représente un groupe hydrocarboné ramifié ou non ramifié de 1 à 3 atomes de carbone,
- les radicaux R⁷ représentent chacun indépendamment les uns des autres un groupe alkylène ramifié ou non ramifié de 2 à 4 atomes de carbone, et
- R⁸ représente un groupe alkylène ramifié ou non ramifié de 1 à 4 atomes de carbone,
les atomes d'hydrogène dans les radicaux R⁶, R⁷ et R⁸ pouvant à chaque fois également être remplacés en totalité ou en partie par fluor.

2. Liquide ionique selon la revendication 1, **caractérisé en ce que** le cation est au moins un choisi dans le groupe des ions de métaux alcalins et des ions de métaux alcalino-terreux.

3. Liquide ionique selon l'une quelconque des revendications 1 à 2, **caractérisé en ce que** R⁶ est un groupe méthyle.

4. Liquide ionique selon l'une quelconque des revendications 1 à 3, **caractérisé en ce qu'**au moins 50 % des groupes R⁷ sont des groupes 1,2-éthylène.

5. Liquide ionique selon l'une quelconque des revendications 1 à 4, **caractérisé en ce que** x représente 3 à 5.

6. Liquide ionique selon l'une quelconque des revendications 1 à 5, **caractérisé en ce que** R⁸ est un groupe méthylène -CH₂-.

7. Liquide ionique selon la revendication 1, **caractérisé en ce que** le cation est Li⁺, Na⁺ ou K⁺, et l'anion est H₃CO-(-CH₂-CH₂-O)₃-CH₂-COO⁻.

8. Procédé de fabrication d'un liquide ionique selon la revendication 1, **caractérisé en ce que**
- lors d'une première étape de réaction, un éther monoalkylique de polyoxyalkylène R⁶O-(R⁷-O-)ₓ-H (IV) est mis en réaction avec un acide halogénocarboxylique de formule générale Hal-R⁸-COOH, Hal représentant F, Cl, Br ou I, pour former l'acide carboxylique R⁶O-(R⁷-O-)ₓ-R⁸-COOH (V), et
- lors d'une deuxième étape de réaction, l'acide carboxylique formé R⁶O-(R⁷-O-)ₓ-R⁸-COOH (V) est neutralisé avec un base X(OH)ₙ ou [X-nH] avec formation du liquide ionique 1/n[Xⁿ⁺][R⁶O-(R⁷-O-)ₓ-R⁸-COO⁻],
à condition que le solvant utilisé et/ou le solvant formé pendant la réaction soient chacun entièrement séparés.

9. Procédé selon la revendication 8, **caractérisé en ce que** l'acide halogénocarboxylique est l'acide chloroacétique.

10. Utilisation de liquides ioniques selon l'une quelconque des revendications 1 à 7 en tant que solvants.

11. Utilisation selon la revendication 10, **caractérisée en ce que** le solvant est utilisé pour la réalisation de réactions chimiques.

12. Utilisation selon la revendication 10, **caractérisée en ce que** le solvant est utilisé pour la dissolution de polymères naturels et synthétiques.

13. Utilisation selon la revendication 10, **caractérisée en ce que** le solvant est utilisé pour des processus électrochimiques.

14. Utilisation selon la revendication 10, **caractérisée en ce que** le solvant est utilisé pour la fabrication et la stabilisation de nanoparticules.

15. Utilisation de liquides ioniques selon l'une quelconque des revendications 1 à 7 en tant qu'agent d'extraction pour l'extraction liquide-liquide.

16. Utilisation de liquides ioniques selon l'une quelconque des revendications 1 à 7 en tant qu'électrolytes ou composants d'électrolytes non aqueux dans des systèmes de stockage d'énergie et des systèmes de transformation d'énergie.

17. Utilisation de liquides ioniques selon l'une quelconque des revendications 1 à 7 en tant que lubrifiant haute température.
